# EUROPEAN PATENT APPLICATION

(11) **EP 4 183 313 A1**
(43) Date of publication of application: **24.05.2023**
(21) Application number: 22204275.6
(22) Date of filing: 28.10.2022
(51) Int. Cl.: A61B 1/00, A61B 34/30

(54) **SYSTEM AND DEVICE FOR ENDOSCOPE SURGERY ROBOT**

(30) Priority: 22.11.2021 KR 20210161288; 07.12.2021 KR 20210173659; 26.10.2022 KR 20220139714
(71) Applicant: ROEN Surgical, Inc., Daejeon 34051 (KR)
(72) Inventor: CHEON, Byung Sik, 34125 Daejeon (KR); KWON, Dong Soo, 34051 Daejeon (KR)
(74) Representative: AWA Sweden AB

(57) **Abstract**

An endoscope surgical robot system according to an exemplary embodiment may include an endoscope apparatus having an insertion tube and an endoscope camera to monitor an end image of the insertion tube, a driving module connected to the endoscope apparatus and configured to perform pitch rotation or roll rotation of the insertion tube, an operation module configured to be rotated to generate an input signal required for pitch rotation or roll rotation of the endoscope apparatus by a user, and a controller to control operation of the driving module based on the input signal generated by the operation module.

## Description

### CROSS-REFERENCE TO RELATED APPLICATION

This present application claims the benefit of priority to Korean Patent Application No. 10-2021-0161288 entitled "ENDOSCOPE SURGERY ROBOT SYSTEM AND IMAGE CORRECTION METHOD THEREOF," filed on November 22, 2021, Korean Patent Application No. 10-2021-0173659 entitled "ENDOSCOPE SURGERY ROBOT SYSTEM," filed on December 7, 2021 and Korean Patent Application No. 10-2022-0139714 entitled "ENDOSCOPE SURGERY ROBOT DEVICE AND ENDOSCOPE SURGERY ROBOT SYSTEM," filed on October 26, 2022, in the Korean Intellectual Property Office, the entire disclosures of which are incorporated herein by reference.

### FIELD

The present disclosure relates to an endoscope surgical robot control device and an endoscope surgery robot system.

### BACKGROUND

Endoscopic surgery refers to a surgical technique that inserts an instrument with a micro-camera attached thereto into a body to remove an appendage or operate on a specific area. A small incision is used to speed up recovery and give a cosmetic benefit. In recent years, endoscopic surgery is performed using a robot.

An endoscope surgical robot includes a master device which is manipulated by a user to generate and transmit an input signal required to drive an endoscopic apparatus and a slave device, which receives a signal from the master device to directly apply manipulation required for the endoscopic apparatus.

Generally, a master device for an endoscope surgical robot may include a) a translation part which receives translation manipulation from the user to input translation operation of an insertion tube, b) a roll rotation part which is connected to the translation part and receives joint rotation manipulation around a roll axis from the user to input a roll rotation operation of the insertion tube, c) a pitch rotation part which is connected to the roll rotation part and receives a joint rotation manipulation around a pitch axis from the user to input pitch rotation operation of the insertion tube, and d) a yaw rotation part which is connected to the pitch rotation part and receives a joint rotation manipulation around a yaw axis from the user to input yaw rotation operation of the insertion tube.

That is, the master device for the endoscope surgical robot of the related art has a structure in which a control handle to be grasped by a user is connected to the yaw rotation part, the yaw rotation part is connected to the pitch rotation part, and the pitch rotation part is rotatably connected from the roll rotation part.

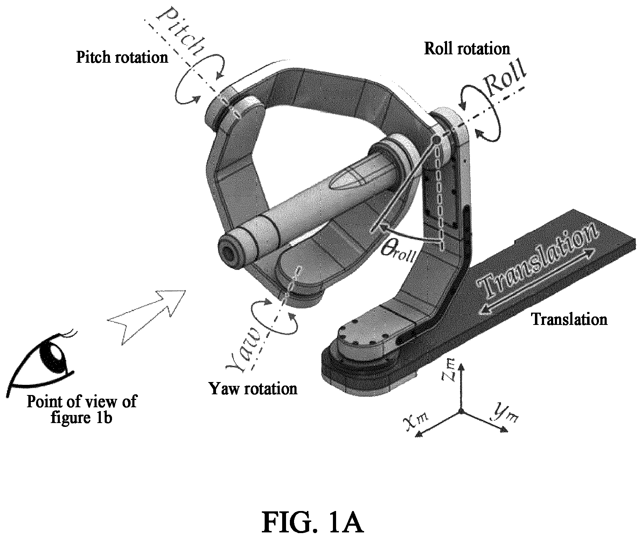

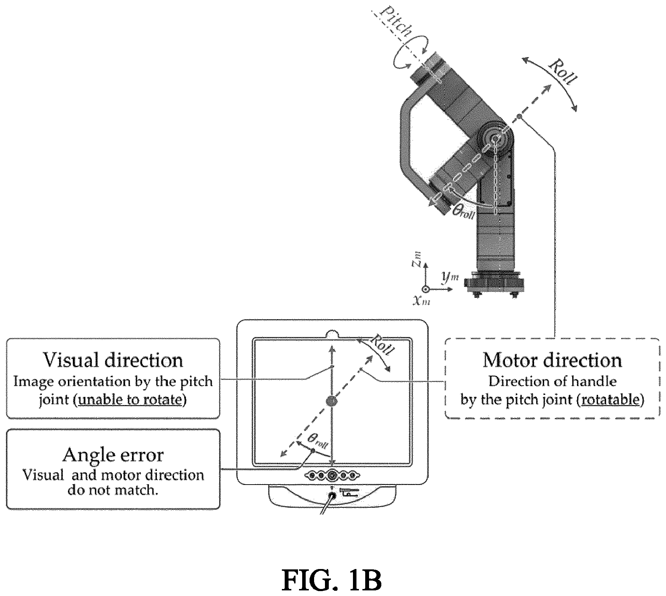

According to this structure, when the user manipulates the master device while watching a display, if the user rotates the roll rotation part for the roll rotation of the insertion tube, as illustrated in FIGS. 1A and 1B, the pitch rotation part and the yaw rotation part rotate around the roll rotation axis according to the rotation of the roll rotation part so that the pitch rotation part and the yaw rotation part also rotate.

FIG. 1A illustrates a master device for an endoscope surgical robot of the related art. In the master device for an endoscope surgical robot of the related art, a part of controlling translation movement of the endoscopic apparatus is connected to a part of controlling roll rotation of the endoscopic apparatus and the part of controlling roll rotation of the endoscopic apparatus is connected to a part of controlling pitch rotation of the endoscopic apparatus, and the part of controlling pitch rotation of the endoscopic apparatus is connected to a part of controlling yaw rotation of the endoscopic apparatus, and the part of controlling yow rotation of the endoscopic apparatus is connected to a handle grasped by a user.

FIG. 1B is a view for explaining inconsistency of a screen displayed to an operator and an actual manipulation direction in a master device for an endoscope surgical robot of the related art.

As illustrated in FIG. 1B, when the user performs the pitch rotation after performing roll rotation, the user moves the operation module to two o'clock and 8 o'clock directions, but the screen moves to 12 o'clock and 6 o'clock directions on the display. This causes an inconsistency between the user's operation direction and a screen direction on the display making intuitive operation difficult.

That is, when an image output to the display or a coordinate system thereof is fixed, when a user manipulates the master device while watching the display and performs a pitch rotation operation or a yaw rotation operation after the roll rotation operation, a direction of the actual pitch rotation axis of the master device is different from a direction of a pitch rotation axis or a yaw rotation axis on the display making it difficult for intuitive operation.

The above-described Background was experienced by the inventor during the process of deriving the present disclosure and is not necessarily a known technology which has been disclosed to the general public prior to this application.

### SUMMARY

There is a need for an endoscope surgical robot control device which solves the inconvenience of operating an endoscope surgical robot system as described above.

An endoscope surgical robot control device according to an exemplary embodiment is an endoscope surgical robot control device that controls the movement of an insertion tube including: a translation movement part configured to control translation movement of the insertion tube in a translation direction; a pitch rotation part in communication with the translation movement part and configured to control pitch rotation of the insertion tube around a pitch rotation axis; and a roll rotation part in communication with the pitch rotation part and configured to control roll rotation of the insertion tube around a roll rotation axis which is parallel with a translation direction and is perpendicular to the pitch rotation axis.

An endoscope surgical robot system according to another exemplary embodiment may include an endoscope apparatus having an insertion tube and an endoscope camera to monitor a surgical procedure where the insertion tube is inserted; a driving module connected to the endoscope apparatus and configured to perform pitch rotation or roll rotation of the insertion tube; the above-described endoscope surgical robot control device; and a controller to control operation of the driving module based on an input signal generated by the endoscope surgical robot control device.

An endoscope surgical robot system according to another exemplary embodiment may include an endoscope apparatus having an insertion tube and an endoscope camera to monitor an end image of the insertion tube; a driving module connected to the endoscope apparatus and configured to perform pitch rotation or roll rotation of the insertion tube; an operation module configured to be rotated to generate an input signal required for pitch rotation or roll rotation of the endoscope apparatus by a user; and a controller to control operation of the driving module based on an input signal generated by the operation module.

The above-described operation module may include a pitch rotation part which rotates a joint along a pitch rotation axis corresponding to a rotation axis of the pitch rotation of the insertion tube and a control handle which is connected to the pitch rotation part to be grasped by the user and is connected to the pitch rotation part to rotate around the roll rotation axis perpendicular to the pitch rotation axis.

Here, the pitch rotation part may include a pitch rotation arm which is configured to rotate around the pitch rotation axis with respect to a member connected to a fixed point of the operation module and a bending arm having one end connected to the pitch rotation arm and the other end extending along the pitch rotation axis to connect the control handle to rotate the joint around the roll rotation axis perpendicular to the pitch rotation axis. The control handle may include a rotation connection part which is connected to the bending arm to rotate around the roll rotation axis and a grasp manipulation part having at least a part which extends along the roll rotation axis to be spaced apart from the rotation connection part to be grasped and manipulated by the user.

Here, the bending arm is connected to the pitch rotation arm to rotate around the rotation axis parallel with the roll rotation axis, the rotation connection part and the bending arm of the control handle are perpendicular to each other regardless of the rotation state therebetween, and the bending arm and the pitch rotation arm are perpendicular to each other regardless of the rotation state therebetween.

Further, the operation module may further include a translation movement part including a sliding guide extending along a direction parallel with the roll rotation axis and a sliding arm having one end which is guided by the sliding guide to be slidable and the other end to which the pitch rotation arm is joint-rotatably connected around the pitch rotation axis.

One end of the sliding arm connected to the sliding guide extends along the pitch rotation axis to be spaced apart from the sliding guide and the other end forms a pitch rotation arm and a joint at a portion which is bent upward perpendicular to the pitch rotation axis and extends and the bending arm extends to be close to the sliding guide along a direction parallel with the pitch rotation axis from a portion connected to the pitch rotation arm.

The sliding arm, the pitch rotation arm, the bending arm, and the control handle have a joint link structure in which the sliding arm, the pitch rotation arm, the bending arm, and the control handle are relatively inwardly perpendicularly bent to be sequentially connected while being spaced apart from one another with a predetermined distance or more.

According to the endoscope surgical robot system according to the exemplary embodiment, even though the user performs the roll rotation on a control handle, a pitch rotation axis of the operation module is consistently maintained and the pitch rotation direction on the image coordinate output on the display and a rotation direction of the actual pitch rotation part are consistently maintained regardless of the roll rotation so that an intuitive controllability may be provided to a user who operates the operation module.

### BRIEF DESCRIPTION OF THE DRAWINGS

The foregoing and other aspects, features, and advantages of the invention, as well as the following detailed description of the embodiments, will be better understood when read in conjunction with the accompanying drawings. For the purpose of illustrating the present disclosure, there is shown in the drawings an exemplary embodiment, it being understood, however, that the present disclosure is not intended to be limited to the details shown because various modifications and structural changes may be made therein without departing from the spirit of the present disclosure and within the scope and range of equivalents of the claims. The use of the same reference numerals or symbols in different drawings indicates similar or identical items.
FIG. 1A illustrates a master device for an endoscope surgical robot of the related art;
FIG. 1B is a view for explaining inconsistency of a screen displayed to an operator and an actual manipulation direction in a master device for an endoscope surgical robot of the related art;
FIG. 2 schematically illustrates a usage environment of an endoscope surgical robot system according to an exemplary embodiment;
FIG. 3 schematically illustrates a configuration of an endoscope surgical robot system according to an exemplary embodiment;
FIG. 4 is a block diagram of an endoscope surgical robot system according to an exemplary embodiment;
FIG. 5 is a perspective view of a first operation module which is an endoscope surgical robot control device according to an exemplary embodiment;
FIG. 6 is a view illustrating an output image of a display part according to the operation of a first operation module according to an exemplary embodiment and an operation configuration of an endoscope apparatus;
FIG. 7 is a view illustrating a configuration of an output image of a display part according to another operation of a first operation module according to an exemplary embodiment;
FIG. 8 is a view illustrating a configuration of an output image of a display part according to still another operation of a first operation module according to an exemplary embodiment;
FIG. 9 is a view schematically illustrating a configuration of an endoscope surgical robot system according to another exemplary embodiment;
FIG. 10 is a perspective view of a second operation module which is an endoscope surgical robot control device according to another exemplary embodiment;
FIG. 11 is a kinematic conceptual view of a second operation module according to another exemplary embodiment;
FIG. 12 is a view schematically illustrating a change in an output image of a display part according to an operation of a second operation module according to another exemplary embodiment;
FIG. 13A is a perspective view of a third operation module which is an endoscope surgical robot control device according to another exemplary embodiment;
FIG. 13B is a view for explaining a handle included in a third operation module according to still another exemplary embodiment in more detail;
FIG. 14 is a view for explaining an endoscope surgical robot control system including an endoscope surgical robot control device according to still another exemplary embodiment;
FIG. 15 is a view for explaining a forceps device controlled by an endoscope surgical robot control system according to still another exemplary embodiment;
FIG. 16 is a view for explaining a laser device controlled by an endoscope surgical robot control system according to still another exemplary embodiment;
FIG. 17A illustrates a front surface of an additional control device included in an endoscope surgical robot control system according to still another exemplary embodiment;
FIG. 17B illustrates a rear surface of an additional control device included in an endoscope surgical robot control system according to still another exemplary embodiment;
FIG. 17C is a view for explaining a joystick of an additional control device according to still another exemplary embodiment;
FIG. 18 is a view schematically illustrating a configuration of an endoscope surgical robot system according to an additional exemplary embodiment;
FIG. 19 is a block diagram of an endoscope surgical robot system according to an additional exemplary embodiment;
FIG. 20 is a perspective view of an operation module according to an additional exemplary embodiment;
FIG. 21 is a view illustrating an output image of a display part according to the operation of an operation module according to an additional exemplary embodiment and an operation configuration of an endoscope apparatus;
FIG. 22 is a flowchart of a method for correcting an output image of an endoscope surgical robot system according to an additional exemplary embodiment;
FIG. 23 is a view illustrating a configuration of an output image of a display part according to an operation of an operation module according to an additional exemplary embodiment;
FIG. 24 is a view illustrating a configuration of an output image of a display part according to an operation of an operation module according to an additional exemplary embodiment.
FIG. 25 is a graph to compare surgery time taken when using a conventional master device for an endoscope surgical robot and surgery time taken when using a master device according to an exemplary embodiment for an endoscope surgical robot.
FIG. 26 is a graph to compare mean number of mistakes occurred when using a conventional master device for an endoscope surgical robot and mean number of mistakes occurred when using a master device according to an exemplary embodiment for an endoscope surgical robot.

### DETAILED DESCRIPTION

Hereinafter, embodiments will be described in detail with reference to the accompanying drawings. The following description is one of various aspects of exemplary embodiments and the following description forms a part of the detailed description of the exemplary embodiment.

In relation to describing one exemplary embodiment, when the detailed description of the relevant known technology or configuration is determined to unnecessarily obscure the gist of the present disclosure, the detailed description may be omitted.

Terms or words used in the present specification and claims should not be interpreted as being limited to typical or dictionary meanings, but should be interpreted as having meanings and concepts which comply with the technical spirit of the endoscope surgical robot system according to the exemplary embodiment, based on the principle that an inventor can appropriately define the concept of the term to describe his/her own invention in the best manner.

Further, it should be understood that the exemplary embodiment described in the specification and the configuration illustrated in the drawings are merely examples of the endoscope surgical robot system according to the exemplary embodiment, but do not represent all the technical spirits of the endoscope surgical robot system according to the exemplary embodiment so that there may be various equivalent and modifications which may be substituted for them at the time of the present application.

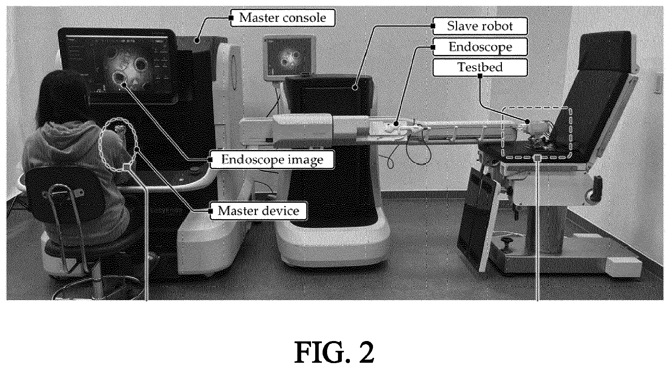

FIG. 2 schematically illustrates a usage environment of an endoscope surgical robot system according to an exemplary embodiment.

FIG. 2 illustrates a test environment in which a test bed is disposed at the right instead of an actual patient, the endoscope is connected to a slave robot and the slave robot is manipulated by a master device (operation module). The endoscope photographs the inside of the test bed while moving in the test bed in accordance with the operation of the master device and the generated image is displayed on a display of a master console. The user controls a movement of the endoscope while watching the display.

Here, the user operates the master device while observing a surgical situation and the movement of the endoscope displayed on a display screen to move the endoscope to be appropriate for a surgical procedure.

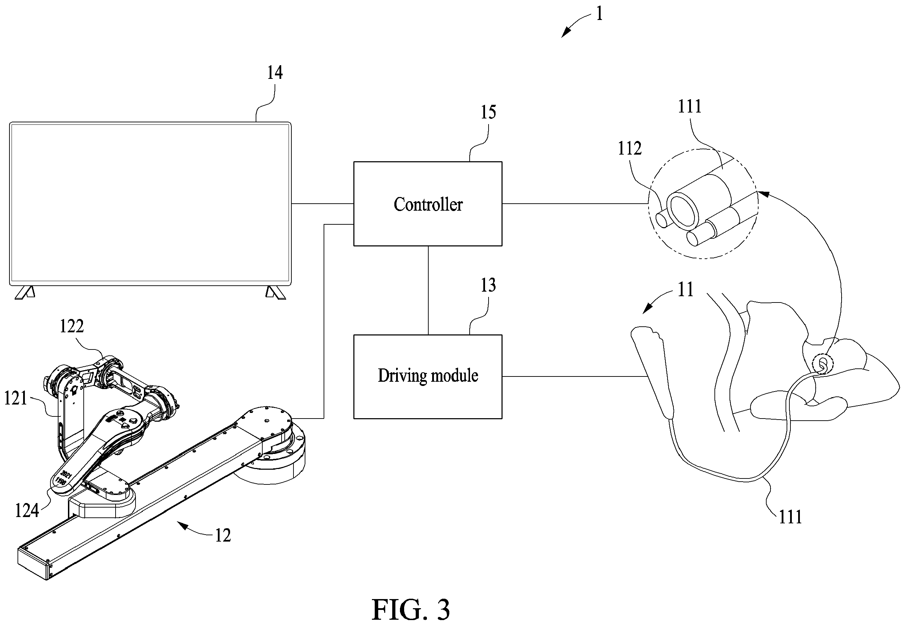

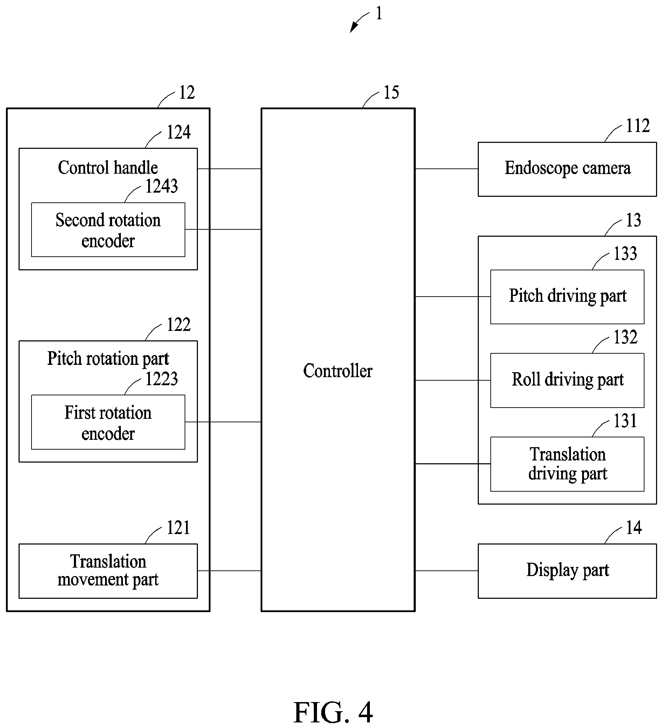

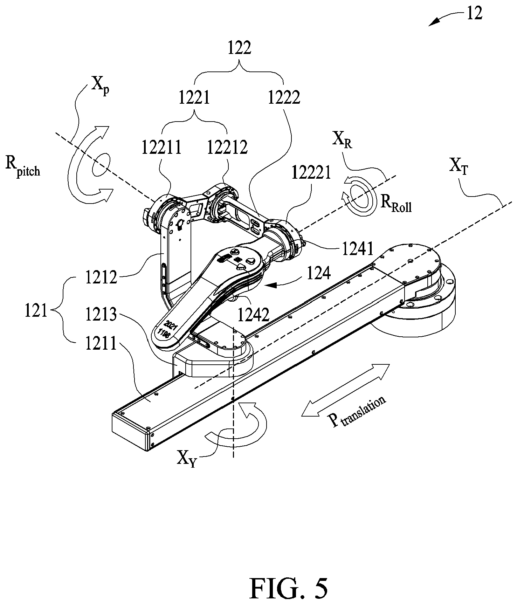

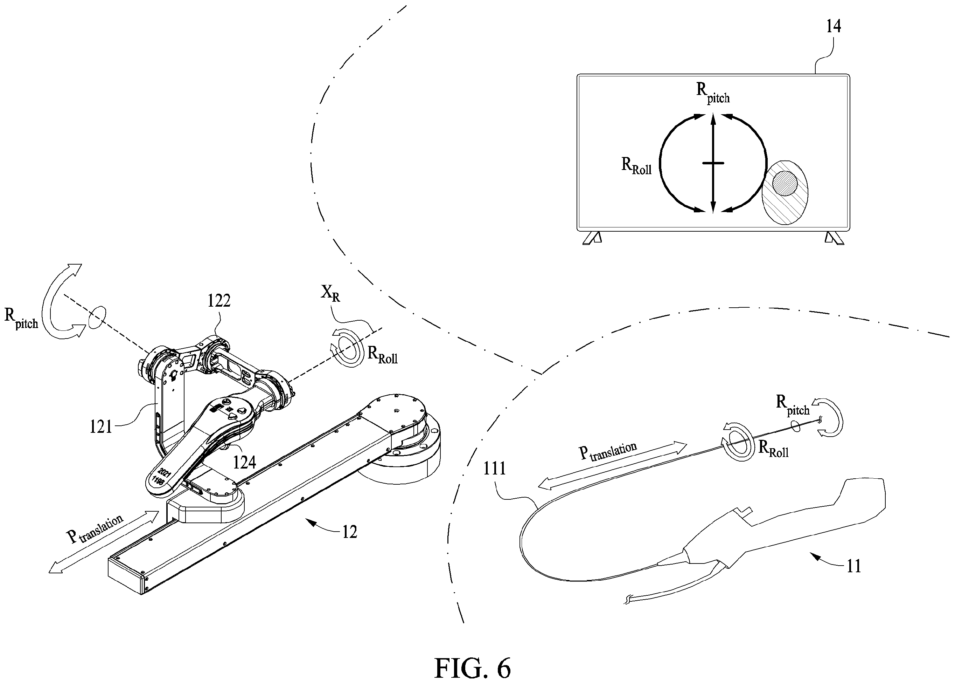

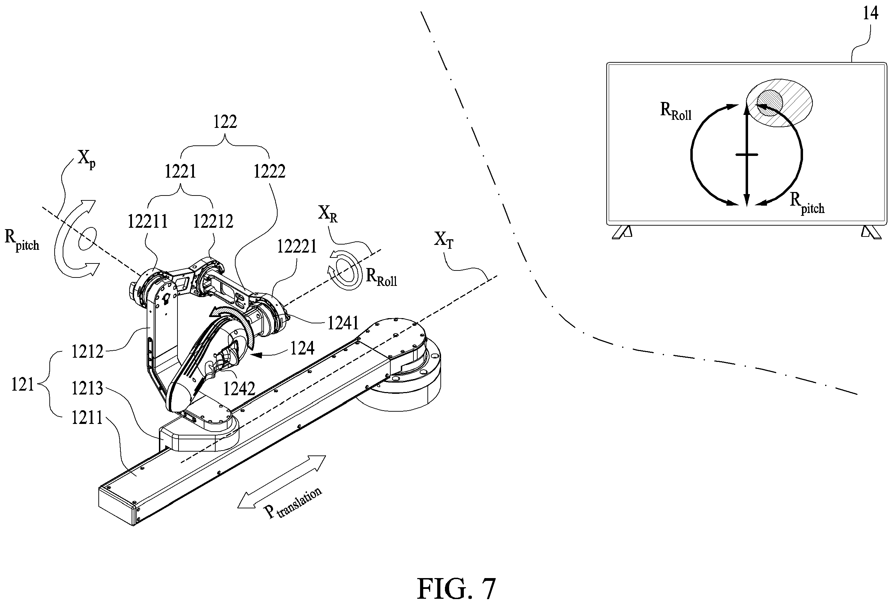

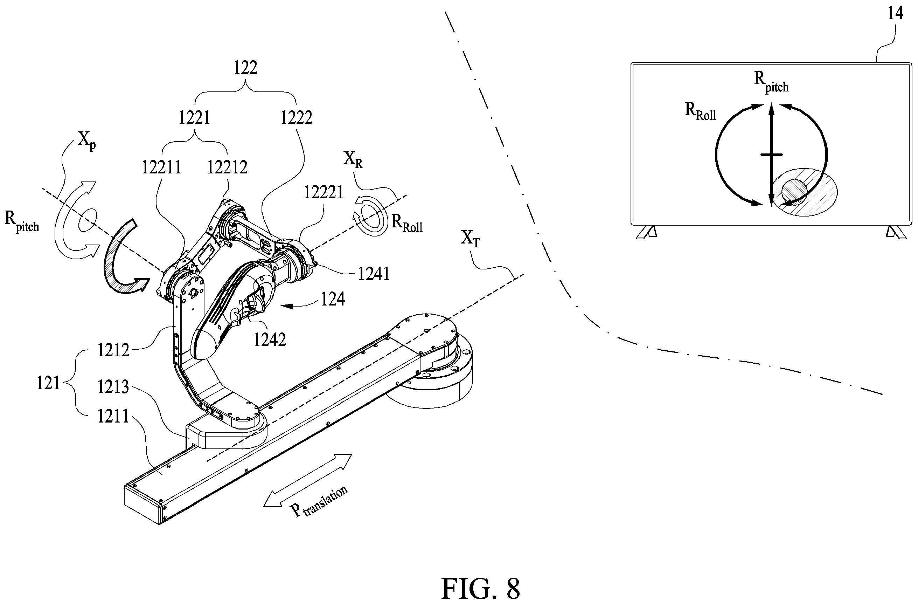

FIG. 3 is a view schematically illustrating a configuration of an endoscope surgical robot system according to an exemplary embodiment. FIG. 4 is a block diagram of an endoscope surgical robot system according to an exemplary embodiment. FIG. 5 is a perspective view of a first operation module which is an endoscope surgical robot control device according to an exemplary embodiment. FIG. 6 is a view illustrating an output image of a display part according to the driving of a first operation module according to an exemplary embodiment and an operation configuration of an endoscope apparatus. FIG. 7 is a view illustrating a configuration of an output image of a display part according to another operation of a first operation module according to an exemplary embodiment. FIG. 8 is a view illustrating a configuration of an output image of a display part according to still another operation of a first operation module according to an exemplary embodiment.

Referring to FIGS. 3 to 8, an endoscope surgical robot of an endoscope surgical robot system 1 according to an exemplary embodiment receives an input signal from a first operation module 12 corresponding to a master device which is manipulated by the user to generate and transmit a required input signal. A slave device which is directly connected to the endoscope apparatus 11 receives an input signal and performs an operation corresponding to the input signal to perform the operation of an insertion tube 111 of the endoscope apparatus 11.

For example, the endoscope surgical robot system 1 may include an endoscope apparatus 11, a driving module 13, a first operation module 12, a display part 14, and a controller 15. The driving module 13 may be configured by various actuators and gears and the controller 15 may include a processor.

The endoscope apparatus 11 may include an insertion tube 111 which is inserted into a body of a patient to perform the translation and rotation and an endoscopic camera 112 which photographs an image at an end portion of the insertion tube 111.

For example, the endoscope apparatus 11 may be connected to the driving module 13 and the driving module 13 is driven to perform the translation and the rotation of the insertion tube 111.

The driving module 13 is connected to the endoscope apparatus 11 to perform the translation and the rotation of the insertion tube 111. For example, the driving module 13 may be a slave device which receives an input signal from the first operation module 12 corresponding to a master device to directly perform the operation required for the endoscope apparatus 11.

For example, the driving module 13 may include a translation driving part 131 which performs translation P_translation of the insertion tube 111 along a translation movement axis X_T with respect to a length direction of the insertion tube 111, a roll driving part 132 which performs roll rotation R_roll around a roll rotation axis X_R parallel with the length direction of the insertion tube 111, and a pitch driving part 133 which performs pitch rotation R_pitch to bend the insertion tube 111 with respect to a pitch rotation axis X_P perpendicular to the roll rotation axis X_R.

The first operation module 12 may be an input device manipulated by the user. For example, the first operation module 12 may be a master device which is manipulated by the user to generate an input signal required for driving the endoscope apparatus 11.

For example, the first operation module 12 may include a translation movement part 121, a pitch rotation part 122, and a control handle 124.

The translation movement part 121 may slidably move along the translation movement axis X_T corresponding to a forward/backward direction of the insertion tube 111 in a state in which the user grasps the control handle 124 and thus, the controller 15 may control the operation of the translation driving part 131 which performs the forward/backward operation of the insertion tube 111 based on a translation movement displacement of the translation movement part 121.

For example, the translation movement part 121 may include a sliding guide 1211 extending along the translation movement axis X_T, a slider 1213 which is guided by the sliding guide 1211 to slide along the translation movement axis X_T, and a sliding arm 1212 which is fixed to the slider 1213 and extends upward.

The sliding arm 1212 may be a connection member which is supported by the pitch rotation part 122 to rotate around the pitch rotation axis X_P. For example, the sliding arm 1212 may extend in a direction perpendicular to the translation movement axis X_T and form a rotatable joint around the pitch rotation axis X_P together with the pitch rotation arm 1221 of the pitch rotation part 122 in an upwardly extending position.

One side of the sliding arm 1212 connected to the slider 1213 may extend to be spaced apart from the sliding guide 1211 along the direction of the pitch rotation axis X_P. Another side of the sliding arm 1212 is connected to the pitch rotation arm 1221 and forms a joint with the pitch rotation arm 1221 in a portion which is bent in a direction perpendicular to the roll rotation axis X_R and the pitch rotation axis X_P.

The first operation module 12 not only performs pitch rotation and roll rotation of the endoscope apparatus 11, but also performs yaw rotation. In this case, as illustrated in FIG. 5, the sliding arm 1212 may rotate around a yaw rotation axis X_Y perpendicular to the roll rotation axis X_R and the pitch rotation axis X_P with respect to the slider 1213. The controller 15 may perform the yaw rotation of the endoscope apparatus 111 based on a rotation direction of the sliding arm 1212 around the yaw rotation axis X_Y and an input signal for rotational displacement.

The pitch rotation part 122 may rotate a joint along the pitch rotation axis X_P corresponding to a rotation axis of the pitch rotation R_pitch of the insertion tube 111 in a state in which the user grasps the control handle 124. Accordingly, the controller 15 may control the operation of the pitch driving part 133 which performs the pitch rotation R_pitch of the insertion tube 111 based on a rotation displacement of the pitch rotation part 122.

The pitch rotation part 122 may include a pitch rotation arm 1221 which is configured to rotate around the pitch rotation axis X_P with respect to the sliding arm 1212, a first rotation encoder 1223 which measures a pitch rotation motion of the pitch rotation arm 1221, and a bending arm 1222 which is connected to the pitch rotation arm 1221 to extend in a direction perpendicular to a length direction of the pitch rotation arm 1221.

The pitch rotation arm 1221 may include a first joint 12211 which is connected to the sliding arm 1212 to rotate around the pitch rotation axis X_P and a second joint 12212 which is connected to the bending arm 1222 to rotate around a rotation axis parallel with the pitch rotation axis X_P. For example, as illustrated in FIGS. 5 to 8, the pitch rotation arm 1221 may be a link member having the first joint 12211 and the second joint 12212 having rotation axes parallel to the pitch rotation axis X_P.

The first rotation encoder 1223 may measure a rotation motion of the pitch rotation arm 1221 around the pitch rotation axis X_P with respect to the sliding arm 1212.

The bending arm 1222 may be a connection link member extending in a direction perpendicular to an extending direction of the pitch rotation arm 1221, that is, a direction parallel with the pitch rotation axis X_P.

For example, the bending arm 1222 may extend from a portion connected to the pitch rotation arm 1221 toward sliding guide 1211 along a direction parallel with the pitch rotation axis X_P. According to this structure, the control handle 124 connected from the bending arm 1222 may maintain a distance adjacent to the sliding guide 1211 with respect to the pitch rotation axis X_P direction (horizontal direction) to advantageously optimize a movement/rotation operation by the control handle 124 or stability in a steady state.

For example, the bending arm 1222 may include a rotation joint 12221 which rotatably supports the control handle 124 around the roll rotation axis X_R perpendicular to the pitch rotation axis X_P in a portion which extends along a direction parallel with the pitch rotation axis X_P from the pitch rotation arm 1221.

For example, the bending arm 1222 may be rotatably connected to the pitch rotation arm 1221 with respect to a rotation axis parallel with the roll rotation axis X_R. In this case, as illustrated in FIGS. 7 and 8, even though the pitch rotation arm 1221 rotates, the bending arm 1222, the control handle 124, and the roll rotation axis X_R which mutually rotates do not rotate to guide the control handle 124 to suppress unnecessary posture or a phase change in a direction excluding the pitch rotation axis X_R.

As another example, it should be noted that the bending arm 1222 may be formed by an integral configuration which does not rotate with respect to the pitch rotation arm 1221.

The control handle 124 is a member which is connected from the pitch rotation part 122 to be grasped by the user and connected to the bending arm 1222 to rotate around the roll rotation axis X_R. For example, the user may grasp the control handle 124 to move or rotate the control handle 124 along any one or more of the translation movement axis X_T, the roll rotation axis X_R, and the pitch rotation axis X_R.

For example, the control handle 124 may include a rotation connection part 1241 which is connected to the bending arm 1222 to be rotatable around the roll rotation axis X_R, a grasp manipulation part 1242 which is connected to the rotation connection part 1241 to be manipulated by being grasped by the user, and a second rotation encoder 1243 which measures the roll rotation motion of the rotation connection part 1241 with respect to a rotation joint 12221 of the bending arm 1222.

For example, the rotation connection part 1241 of the control handle 124 may be disposed to be perpendicular to the bending arm 1222 regardless of a rotation state with respect to the bending arm 1222 and similarly, the bending arm 1222 may maintain to be perpendicular to the pitch rotation arm 1221 regardless of the rotation state thereof.

As illustrated in FIG. 5, the sliding arm 1212, the pitch rotation arm 1221, the bending arm 1222, and the control handle 124 have a joint link structure in which the sliding arm 1212, the pitch rotation arm 1221, the bending arm 1222, and the control handle 124 are relatively inwardly bent to be sequentially connected while being spaced apart from one another with a predetermined distance or more. Therefore, they have an advantageous structure to reduce a moment due to a rotation operation or give stability to each joint portion. Further, it is advantageous to provide an appropriate available space so as not to interfere with the sliding arm 1212, the pitch rotation arm 1221, or the bending arm 1222 while the user performs various movement or rotation operations with the grasped control handle 124.

A grasp manipulation part 1242 may provide an input interface to control operations of the endoscope apparatus 11 or various surgical tools used together.

For example, the grasp manipulation part 1242 may have a shape extending to be spaced apart from a portion connected to the rotation connection part 1241 along the roll rotation axis X_R.

For example, as illustrated in FIG. 5, with respect to a default state in which the grasp manipulation part 1242 does not rotate around the roll rotation axis X_R, a part of the grasp manipulation part 1242 extending along the roll rotation axis X_R may project obliquely downward at a predetermined angle so that it can be easily grasped by the user.

The display part 14 may receive and output an image generated from the endoscope camera 112. For example, the controller 15 may receive an image generated by the endoscope camera 112 and transmit the image to the display part 14.

The controller 15 may receive the input signal generated by the operation of the operation module 12 by the user and control the operation of the driving module 13 based on the input signal to drive the endoscope apparatus 11 in response to the input signal.

For example, the controller 15 may calculate a direction of rotating the pitch rotation arm 1221 around the pitch rotation axis X_P by the user and a rotation displacement based on a signal measured by the first rotation encoder 1223. The controller 15 may perform the pitch rotation R_pitch on the insertion tube 111 by driving the pitch driving part 133 so as to correspond to the calculated rotation direction and rotation displacement of the pitch rotation arm 1221.

For example, the controller 15 may calculate a direction of rotating the roll control handle 124 around the roll rotation axis X_R by the user and a rotation displacement based on a signal measured by the second rotation encoder 1243. The controller 15 may perform the roll rotation R_roll on the insertion tube 111 by driving the roll driving part 132 so as to correspond to the calculated roll rotation direction and roll rotation displacement.

In a master device of the endoscope surgical robot system of the related art, in the case of a configuration in which a joint is formed at a point at which a configuration of a pitch rotation joint rotating to input pitch rotation of an insertion tube with respect to an end portion grasped by the user is prior to a configuration of a roll rotation joint rotating to input rotation of the insertion tube, the entire pitch rotation joint rotates in the roll direction in accordance to the roll rotation operation of the user. Consequently, the pitch rotation axis of the pitch rotation joint rotates so that there is a problem in that a pitch driving direction on an image (endoscope camera image) coordinate output from the display by the user does not match a pitch rotation direction of the master device which is actually manipulated.

According to the endoscope surgical robot system 1 according to the exemplary embodiment, during a process that the user remotely drives the endoscope apparatus 11 by means of the operation module 12 while observing the display part 14, as illustrated in FIGS. 6 and 7, when the control handle 124 rotates around the rotation axis X_R to implement the roll rotation R_roll of the insertion tube 111, the pitch rotation axis X_P of the pitch rotation part 122 maintains a fixed state regardless of the rotation of the control handle 124. Therefore, as illustrated in FIGS. 7 and 8, the pitch rotation R_pitch direction on the output image coordinate and the actual rotation direction of the pitch rotation part 122 are set to always be equal allowing intuitive control by the user.

The endoscope surgical robot system including the above-described first operation module 12 may include an endoscope apparatus including an endoscope surgical robot insertion tube and an endoscope camera 112 which photographs an end image of the insertion tube, a driving module 13 which is connected to the endoscope apparatus to perform pitch rotation or roll rotation of the insertion tube 111, a first operation module 12 which is manipulated by the user to rotate and generate an input signal required for the pitch rotation or the roll rotation of the endoscope apparatus, and a controller 15 which controls the operation of the driving module based on an input signal generated by the first operation module.

Here, the first operation module 12 may include a pitch rotation part 122 which rotates a joint along the pitch rotation axis corresponding to a rotation axis according to the pitch rotation of the insertion tube and a control handle 124 which is connected from the pitch rotation part 122 to be grasped by the user and connected to the pitch rotation part 122 to rotate around the roll rotation axis perpendicular to the pitch rotation axis.

Here, the pitch rotation part 122 may include a pitch rotation arm which is installed to rotate the joint around the pitch rotation axis with respect to a member connected from a fixed point of the first operation module 12 and a bending arm which has one end connected to the pitch rotation arm and the other end extending along the pitch rotation axis to allow the control handle to be connected to rotate the joint around the roll rotation axis perpendicular to the pitch rotation axis.

The control handle may include a rotation connection part which is connected to the bending arm to be rotatable around the roll rotation axis, and a grasp manipulation part which extends at least a small amount to be spaced apart from the rotation connection part along the roll rotation axis to be grasped by the user to be manipulated.

Here, the bending arm is connected to the pitch rotation arm to rotate around the rotation axis parallel with the roll rotation axis, the rotation connection part and the bending arm of the control handle are perpendicular to each other regardless of the rotation state therebetween, and the bending arm and the pitch rotation arm are perpendicular to each other regardless of the rotation state therebetween.

The operation module may further include a translation movement part including a sliding guide extending along a direction parallel with the roll rotation axis and a sliding arm having one side which is guided by the sliding guide to be slidable and the other end to which the pitch rotation arm is joint-rotatably connected around the pitch rotation axis.

Here, one end of a part of the sliding arm which is connected to the slide guide extends along the pitch rotation axis so as to be spaced apart from the sliding guide and the other end is upwardly bent to be perpendicular to the pitch rotation axis to extend and project upward to form a joint with the pitch rotation arm.

The bending arm is characterized by having a shape which extends from a portion connected to the pitch rotation arm along a direction parallel with the pitch rotation axis to be close to the sliding guide.

Here, the sliding arm, the pitch rotation arm, the bending arm, and the control handle are characterized by having a joint link structure in which the sliding arm, the pitch rotation arm, the bending arm, and the control handle are relatively inwardly perpendicularly bent to be sequentially connected while being spaced apart from one another by a predetermined distance.

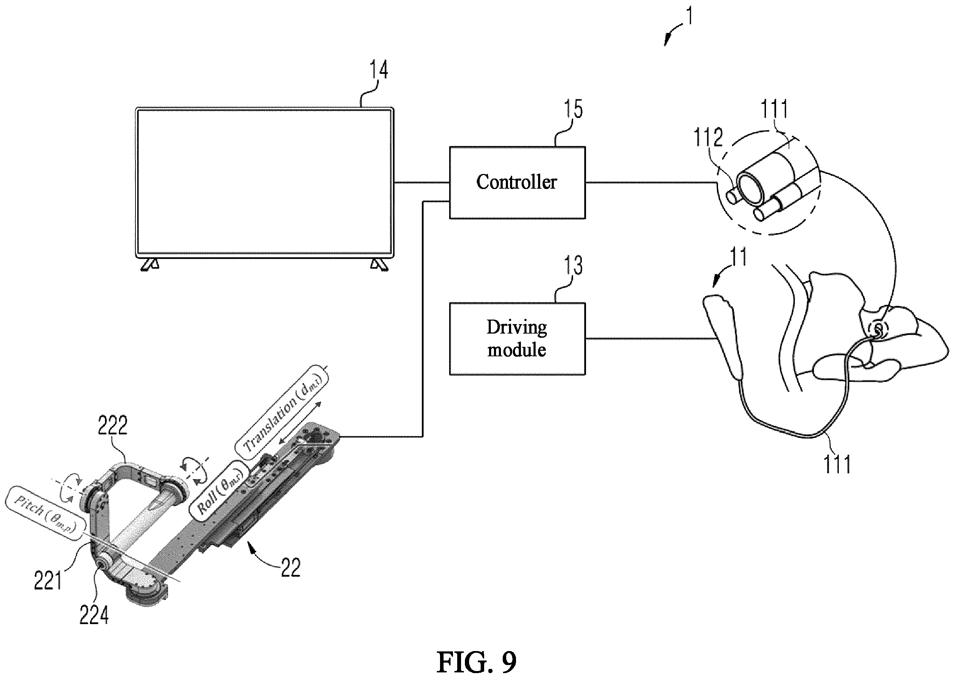

FIG. 9 is a view schematically illustrating a configuration of an endoscope surgical robot system according to another exemplary embodiment.

An endoscope surgical robot system of FIG. 9 includes a second operation module 22 according to another exemplary embodiment and the other configuration excluding the second operation module 22 is the same as that of FIG. 3. Accordingly, the configuration other than the second operation module 22 will be replaced with the description of FIG. 3 and will not be repeated.

The second operation module 22 has a structure similar to that of the first operation module 12 in terms of kinematics and a detailed design and a handle portion to be grasped by the user may be different.

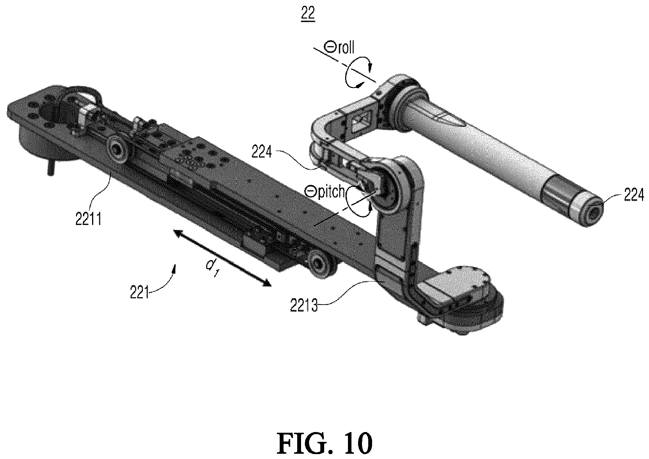

FIG. 10 is a perspective view of a second operation module which is an endoscope surgical robot control device according to another exemplary embodiment.

The second operation module 22 may include a translation movement part 221, a pitch rotation part 222, and a roll rotation part 224. The roll rotation part 224 may correspond to the control handle 124 of the first operation module 12, but may be designed with a different shape.

The translation movement part 221 may move forward and backward to control a forward/backward movement of the insertion tube 111 and may be designed to move a predetermined distance in accordance with the parameters of use for the endoscope apparatus. An endoscope control device may be designed to make a movement distance of the translation movement part 221 equal to the actual movement distance of the insertion tube 111 or the endoscope control device may also be designed to make a predetermined ratio of the movement distance of the translation movement part 221 and the actual movement distance of the insertion tube 111. For example, the endoscope apparatus may be designed such that when the translation movement part 221 moves by d₁, the insertion tube 111 moves by di/k. For example, k may be a fixed value like 10.

In another embodiment, the ratio of the movement distance of the translation movement part 221 and the actual movement distance of the insertion tube 111 is configured to change depending on the distance between the front end of the insertion tube 111 and the inner wall of the organ being detected in the direction in which the front end of the insertion tube 111 is directed. For example, when the distance (d₂) from the front end of the insertion tube 111 inserted into the patient's organ to the inner wall of the organ detected in the direction in which the front end of the insertion tube 111 is directed is 0 to less than 1 cm, k becomes 100, and if the distance d₂ is 1 to less than 5 cm, k becomes 20, and when the distance d₂ is 5 cm or more, k becomes 10. The distance d₂ between the front end of the insertion tube 111 and the inner wall of the organ may be detected by a distance detection sensor disposed on the insertion tube 111. For example, the distance detection sensor includes at least one of an ultrasonic sensor, an infrared sensor, etc. In another embodiment, k may be determined to be inversely proportional to the distance d₂.

The k value may be automatically adjusted according to the environment and conditions that the endoscope surgical robot will be used, or may be manually adjusted according to the user's desired ratio or sensitivity.

The pitch rotation part 222 is connected to the translation movement part 221 and may be configured to rotate around the pitch rotation axis to control the pitch rotation of the insertion tube 111.

The torque required for rotation of the pitch rotation part 222 may be configured to change according to the distance between the side of the insertion tube 111 and the inner wall of the organ. For example, the closer the distance between the inner wall of the organ in which the insertion tube 111 is disposed and the side of the insertion tube 111, the greater the torque required for rotation of the pitch rotation part 222. The closer the inner wall of the organ is to the side of the insertion tube 111, the greater the likelihood of colliding with the inner wall of the organ during a pitch rotation, so it may be necessary to adjust the manipulation sensation of the operator.

The roll rotation part 224 is connected to the pitch rotation part 222 and may be configured to rotate around the roll rotation axis to control the roll rotation of the insertion tube 111. In a default state of the second operation module 22, the roll rotation axis may be designed to be parallel to a translation direction and is perpendicular to the pitch rotation axis. Here, the default state may refer to a state in which the user does not apply a force to the second operation module 22.

In addition, the torque required for rotation of the roll rotation part 224 may be configured to change according to the curvature of the insertion tube 111. For example, as the curvature of the insertion tube 111 increases, the torque required to rotate the roll rotation part 224 increases. If the insertion tube 111 is bent along the path of the organ, the master device can alert the operator that the insertion tube 111 is in a bent state by adjusting the operator's sense of manipulation when the roll rotation part 224 rotates.

The roll rotation part 224 may be designed to be symmetrical with respect to the roll rotation axis.

The translation movement part 221 may be in direct communication with the pitch rotation part 222 and the pitch rotation part 222 may be in direct communication with the roll rotation part 224. Further, the movement of the translation movement part 221, the movement of the pitch rotation part 222, and the movement of the roll rotation part 224 may be independently controlled from one another.

The translation movement part 221 may include a sliding guide 2211 and a sliding arm 2213 extending along the translation direction. Here, one end of the sliding arm 2213 may be configured to slide along the sliding guide 2211 and the other end of the sliding arm 2213 extends upward to be connected to the pitch rotation part 222.

The other end of the sliding arm 2213 extending upward may be designed to be connected to be fixed to one end of the sliding arm 2213 so as not to rotate.

In another exemplary embodiment, the other end of the sliding arm 2213 extending upward may be rotatably connected to one end of the sliding arm 2213 and in this case, the sliding arm 2213 may be designed to rotate around the yaw rotation axis perpendicular to the pitch rotation axis and the roll rotation axis.

In this case, the user may also control the yaw rotation axis of the insertion tube 111.

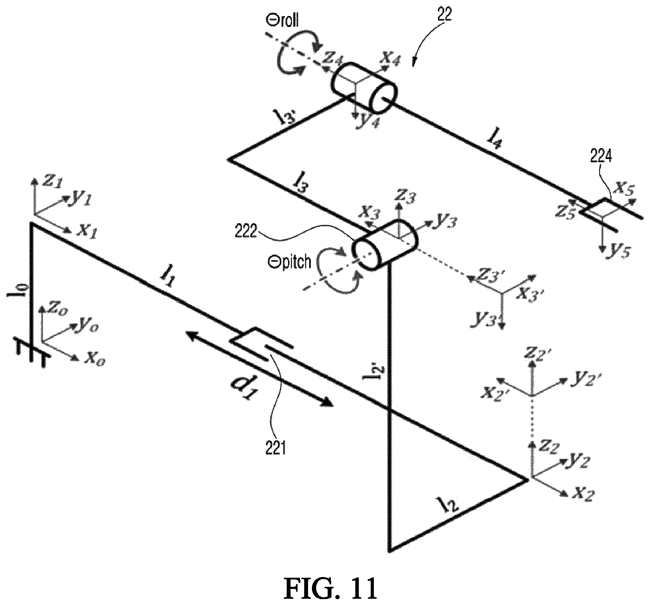

FIG. 11 is a kinematic conceptual view of a second operation module according to another exemplary embodiment.

As illustrated in FIG. 11, from the viewpoint of the kinematic conceptual view of the endoscope surgical robot control device, the translation movement part 221, the pitch rotation part 222 and the roll rotation part 224 may be connected in this order.

In the operation module of the related art illustrated in FIGS. 1A and 1B, a part of controlling the translation motion is directly connected to a part of controlling the roll rotation of the endoscope apparatus, and a part of controlling the roll rotation of the endoscope apparatus is directly connected to a part of controlling the pitch rotation of the endoscope apparatus. In contrast, in the endoscope surgical robot control device according to the present disclosure, a part of controlling the translation movement is directly connected to the part of controlling the pitch rotation, and the part of controlling the pitch rotation is directly connected to the part of controlling the roll rotation, which is substantially different in the connection order from the related art.

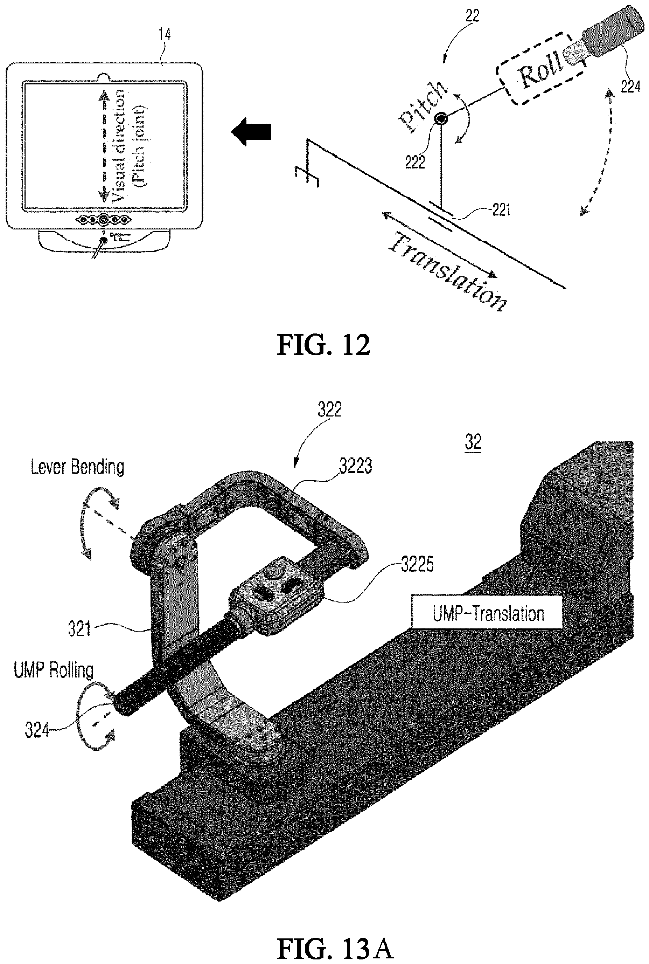

FIG. 12 is a view schematically illustrating a change in an output image of a display part according to an operation of a second operation module according to another exemplary embodiment.

As illustrated in FIG. 12, after performing the roll rotation by the user, the manipulation direction to perform the pitch rotation may match a movement direction (visual direction) displayed on the display. By doing this, the experience that a screen observed by the user intuitively matches the manipulation performed by the user is provided giving a more accurate and efficient surgical environment.

FIG. 13A is a perspective view of a third operation module which is an endoscope surgical robot control device according to another exemplary embodiment.

The third operation module 32 may include a translation movement part 321, a pitch rotation part 322, and a roll rotation part 324.

The pitch rotation part 322 of the third operation module 32 according to still another exemplary embodiment of FIG. 13A is connected to a sliding arm of the translation movement part 321 and has a pitch rotation arm 3223 and a handle 3225.

The pitch rotation arm 3223 may be configured to be connected to the sliding arm of the translation movement part 321 to rotate around the pitch rotation axis.

The handle 3225 of the pitch rotation part 322 includes a first end and a second end and the first end of the handle 3225 may be connected to the pitch rotation arm 3223 and the second end of the handle 3225 may be connected to the roll rotation part 324.

The roll rotation part 324 may include a roll rotation arm connected to the handle 3225 and configured to rotate around the roll rotation arm (a protrusion to be grasped by the user).

A connection part connected to the pitch rotation arm 3223 may be disposed to be close to the first end of the handle 3225 and a joystick for fine adjustment of the insertion tube 111 may be disposed to be close to the second end of the handle 3225.

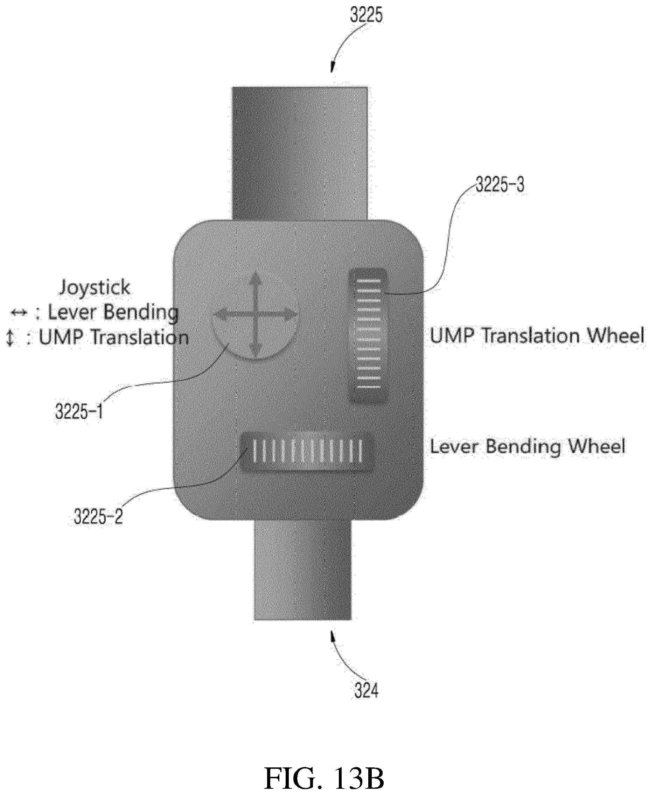

FIG. 13B is a view for explaining a handle included in a third operation module according to still another exemplary embodiment in more detail.

The handle 3225 of FIG. 13B may include a joystick 3225-1 which moves in at least four directions to control the fine translation movement of the insertion tube 111 and the fine pitch rotation of the insertion tube.

Up-down movement of the joystick 3225-1 controls the fine translation movement and left-right movement of the joystick controls the fine pitch rotation.

Further, the up and down direction of the joystick 3225-1 may be parallel with the roll rotation axis and the left and right direction of the joystick 3225-1 may be parallel with the pitch rotation axis.

Further, the handle 3225 may include a first fine adjustment wheel 3225-3 configured to control fine translation movement of the insertion tube 111 and a second fine adjustment wheel 3225-2 configured to control fine pitch rotation of the insertion tube 111. Here, the wheel may be replaced with a lever or a slider, and the wheel in the below may be replaced with a lever or a slider.

The fine adjustment wheels 3225-2 and 3225-3 may be designed to control a finer motion than the joystick.

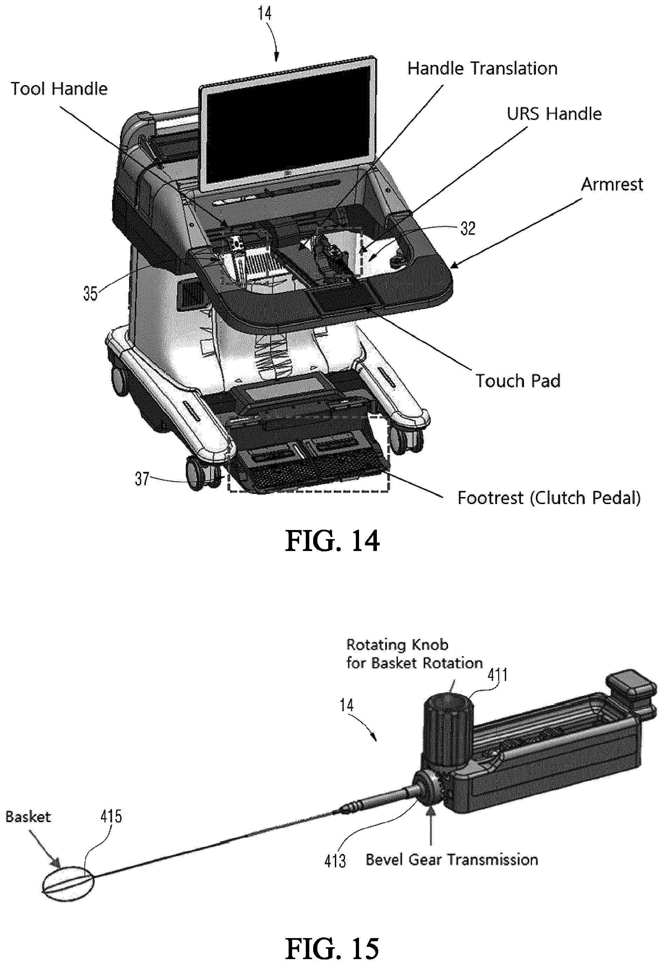

FIG. 14 is a view for explaining an endoscope surgical robot control system including an endoscope surgical robot control device according to still another exemplary embodiment.

An endoscope surgical robot control system of FIG. 14 may further include an additional control device 35 and a clutch pedal 37 in addition to a third operation module 32 which controls the insertion tube 111. The clutch pedal 37 may be used for manipulation of the endoscope apparatus using a foot of the user. The clutch pedal 37 may be used for various functions, and in one embodiment, the clutch pedal 37 may function as a switch that transmits manipulation input signals such as translation, pitch, and roll motion of the third operation module to the driving module.

In another embodiment, the clutch pedal 37 may also be used as a master foot clutch, laser foot clutch, and clutch for irrigation and suction.

In another embodiment, the clutch pedal 37 may be used as an operation device for controlling the laser device, and the control function of the clutch pedal 37 may be selected and changed according to a user's setting.

The additional control device 35 may be used to control a forceps and/or a laser device used during the endoscope surgery and the user may control the third operation module 32 with one hand and control the additional control device 35 with the other hand.

The additional control device 35 may be used to control the forceps device or the laser device to be described below.

In another embodiment, the third operation module 32 and the additional control device 35 may be operated to move left and right.

FIG. 15 is a view for explaining a forceps device controlled by an endoscope surgical robot control system according to still another exemplary embodiment.

When the additional control device 35 is used to control the forceps device, the additional control device 35 may also be referred to as a forceps controller.

In this case, the endoscope surgical robot control device may further include a forceps controller to control forceps connected through a link within the insertion tube and the forceps controller may be configured to control translation movement of the forceps and an open and close operation of the forceps.

When the additional control device 35 is used to control a basket device, the additional control device 35 may also be referred to as a basket controller.

In this case, the endoscope surgical robot control device may further include a basket controller to control a basket connected through a link within the insertion tube 111.

The basket controller may include a translation actuator configured to control translation movement of the basket and an open and close actuator configured to control an open and close operation of the basket.

The basket device 41 controlled by the basket controller may include a basket rotation controller 411, a bevel gear transmission 413, and a basket 415. The basket rotation controller 411 moves to rotate the basket and may perform this by the bevel gear transmission 413.

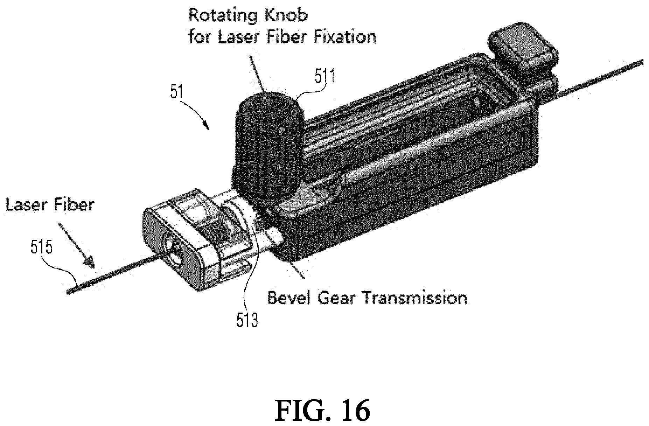

FIG. 16 is a view for explaining a laser device controlled by an endoscope surgical robot control system according to still another exemplary embodiment.

When the additional control device 35 is used to control the laser device, the additional control device 35 may also be referred to as a laser controller.

In this case, the endoscope surgical robot control device may further include a laser controller to control the laser generation device connected through a link within the insertion tube 111.

The laser controller may be configured to control translation movement and a laser generation operation of the laser generation device.

The laser device 51 controlled by the laser controller may include a laser fiber rotation controller 511, a bevel gear transmission 513, and a laser fiber 515. The laser fiber rotation controller 511 can fix the laser fiber not to move.

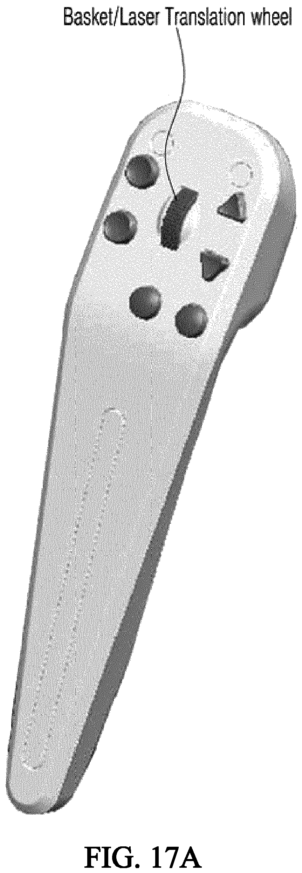

FIG. 17A illustrates a front surface of an additional control device included in an endoscope surgical robot control system according to still another exemplary embodiment.

Various buttons and wheels may be disposed on the front surface of the additional control device 35 and for example, a wheel or a direction key for translation movement of the basket or the laser fiber, a button for rotation of the basket or the laser fiber, and a button to open or close the basket may be disposed.

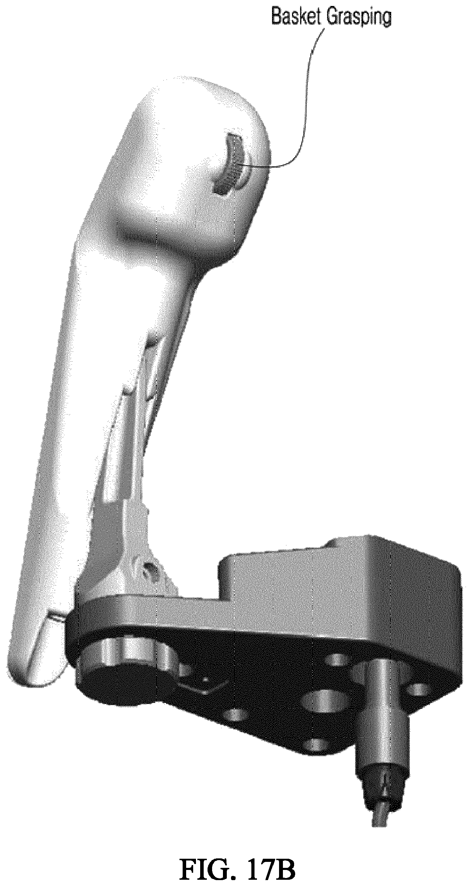

FIG. 17B illustrates a rear surface of an additional control device included in an endoscope surgical robot control system according to still another exemplary embodiment.

Another wheel may be disposed on a rear surface of the additional control device 35 and this wheel may be used for an operation wheel to open and close the basket.

In another embodiment, the wheel on the rear of the additional control device 35 may be used to perform various functions, such as operation for fixing and releasing the laser fiber, operation of the laser, operation of the flow rate of the irrigation/suction pump, etc.

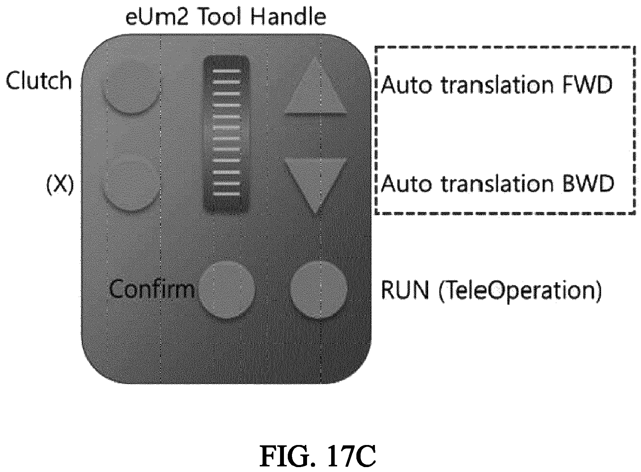

FIG. 17C is a view for explaining a joystick of an additional control device according to still another exemplary embodiment.

The joystick of the additional control device 35 may include a direction key to automatically position the basket or the laser fiber. Further, the additional control device 35 may include a RUN button for operating the laser, a manipulator such as basket wheel, a lever or a slider for opening and closing the basket, and a Confirm button to confirm a user control instruction and may further include a button customized by a user to perform an arbitrary instruction.

Meanwhile, the additional control device 35 of FIG. 17C is only an example, and the functions, positions, and numbers of buttons, wheels, and direction keys may be changed according to usage examples.

Buttons and manipulators of the additional control device 35 may be configured for irrigation control via the insertion module 111, laser operation, position control of the access sheath, etc. and these configurations can be selected and changed by a user.

Meanwhile, the first operation module 12, the second operation module 22, the third operation module 32, and the additional control device 35 may include a feedback function related to the performance of the above-described function. Here, the feedback means may include at least one of vibration, resistance, sound, and light.

According to the above-described exemplary embodiments, the design of the operation module is changed to solve the inconsistency between the displayed image and the manipulated operation.

Hereinafter, another additional method for solving the inconsistency by adjusting a displayed image, instead of changing a design of the operation module will be described.

The additional exemplary embodiment provides an endoscope surgical robot system and a method for correcting an output image using the same.

An endoscope surgical robot system according to the additional exemplary embodiment may include an endoscope apparatus including an insertion tube and an endoscope camera which photographs an end image of the insertion tube, a driving module connected to the endoscope apparatus to perform pitch rotation or roll rotation of the insertion tube, an operation module which is rotated by the user to generate an input signal required for the pitch rotation or the roll rotation of the endoscope apparatus, a controller which controls an operation of the driving module based on an input signal generated by the operation module, and a display part which receives and outputs an image generated by the endoscope camera, and when an input signal for the roll rotation of the insertion tube is formed by the operation module, the controller may rotate the image generated by the endoscope camera in accordance with a rotation direction and a rotation displacement according to the roll rotation.

The operation module may include a control handle grasped by the user, a pitch rotation part which is connected to the control handle and rotates a joint along the pitch rotation axis corresponding to the rotation axis according to the pitch rotation of the insertion tube, and a roll rotation part which is connected to the control handle and rotates a joint along a roll rotation axis which corresponds to a rotation axis according to the roll rotation and is perpendicular to the pitch rotation axis. The operation module may have a joint link structure forming a joint in which a joint part of the pitch rotation part is closer to the j oint link structure that a j oint part of the roll rotation part.

The roll rotation part may include a roll rotation arm having one end connected to a member connected to a fixed point of the operation module to rotate a joint around the roll rotation axis and the other end connected to the pitch rotation part to rotate a joint around the pitch rotation axis and a rotation encoder which measures a direction in which the roll rotation arm rotates around the roll rotation axis and a rotation displacement. The controller may rotate and correct an image input from the endoscope camera in proportion to a rotation direction and the rotation displacement of the roll rotation arm based on a signal measured by the rotation encoder, and output the rotated and corrected image on the display part.

The operation module may further include a sliding guide which is connected to the control handle and extends along a direction parallel with the roll rotation axis and a translation movement part having a sliding arm which has one end of which is guided by the sliding guide to be slidable and the other end of which extends upward to be perpendicular to a direction of the roll rotation axis to rotate the roll rotation part around the roll rotation axis.

A method for correcting an output image of an endoscope surgical robot system according to an exemplary embodiment including an operation module including a control handle grasped by the user, a pitch rotation part which is connected to the control handle and has a joint link structure which rotates a joint along the pitch rotation axis corresponding to a rotation axis according to the pitch rotation of the insertion tube of the endoscope apparatus, and a roll rotation part which is connected with a joint link structure following the pitch rotation part with respect to the control handle and rotates a joint along a roll rotation axis which corresponds to a rotation axis according to the roll rotation of the insertion tube and is perpendicular to the pitch rotation axis, a display part which outputs an image generated by an endoscope camera which photographs an end portion of the insertion tube of the endoscope apparatus, and a controller which controls roll rotation or pitch rotation of the insertion tube of the endoscope apparatus based on an input signal formed by rotating the operation module by the user may include: a step of sensing roll rotation of the roll rotation part with respect to the roll rotation axis, by the controller; a step of calculating roll rotation movement information including a direction that the user rotates the roll rotation part and a rotation displacement based on the sensed roll rotation by the controller, a step of rotating and correcting an image generated by the endoscope camera based on the calculated roll rotation movement information by the controller, and a step of transmitting and outputting the corrected image to the display part by the controller.

The roll rotation part may include a roll rotation arm having one end connected to a member connected to a fixed point of the operation module to rotate a joint around the roll rotation axis and the other end connected to the pitch rotation part to rotate a joint around the pitch rotation axis and a rotation encoder which measures a direction in which the roll rotation arm rotates around the roll rotation axis and a rotation displacement. In the step of calculating roll rotation movement information, the controller may measure a rotation direction in which the user rotates the roll rotation arm of the roll rotation part around the roll rotation axis and a rotation displacement based on a signal measured by the rotation encoder to calculate the roll rotation movement information.

In the step of rotating and correcting an image, the controller may rotate the image generated by the endoscope camera in the same rotation direction and at the same angle as the rotation direction and the rotation displacement of the roll rotation part included in the roll rotation movement information.

According to the endoscope surgical robot system according to the additional exemplary embodiment and the method for correcting an output image using the same, as the user rotates the roll rotation part by the control handle, when the pitch rotation axis of the pitch rotation part rotates together, the image of the endoscope camera output on the display rotates in accordance with the rotation movement of the roll rotation so that a pitch rotation direction on the image coordinate output on the display is set to be identical to the actual rotation direction of the pitch rotation part. Accordingly, an intuitive controllability may be provided to the user who manipulates the operation module.

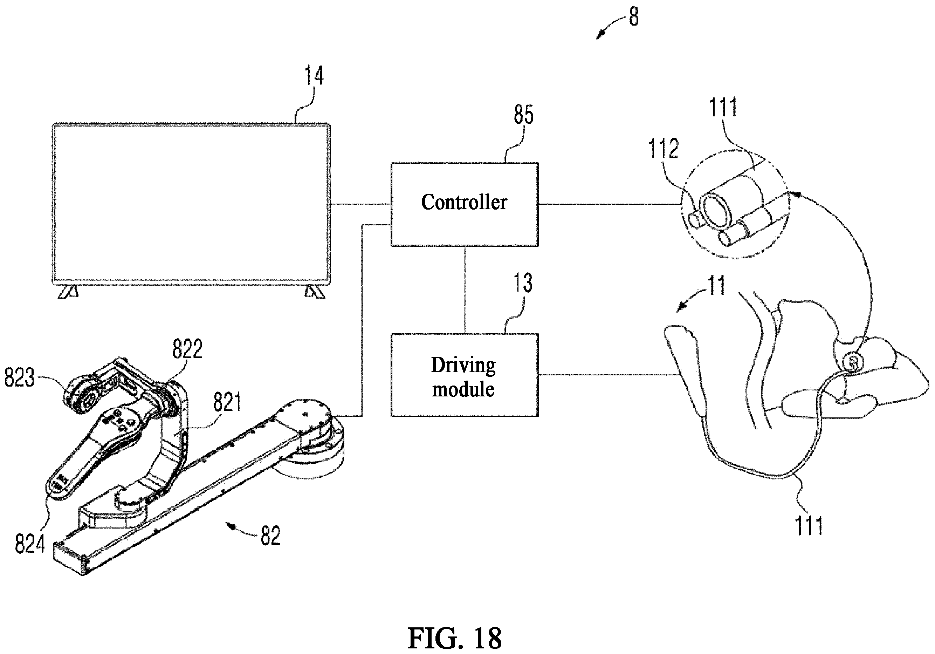

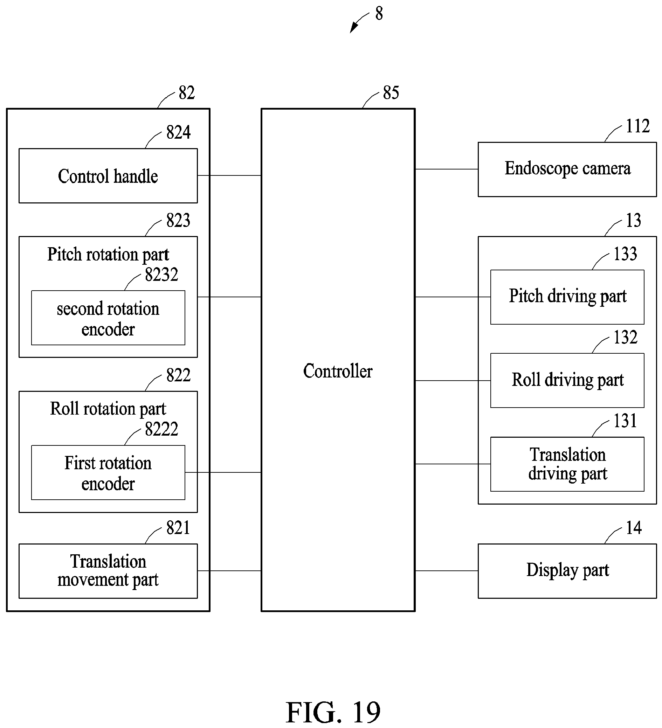

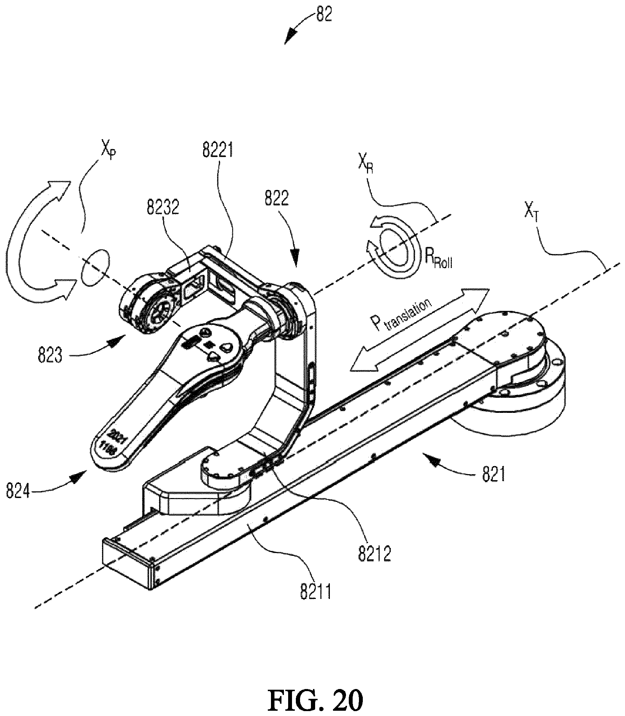

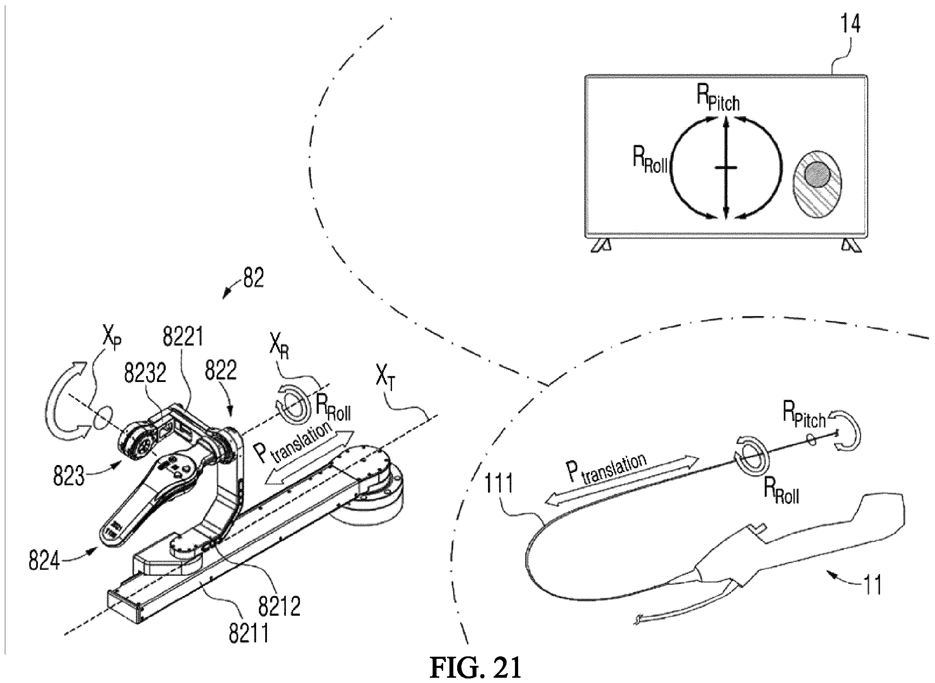

FIG. 18 is a view schematically illustrating a configuration of an endoscope surgical robot system according to an exemplary embodiment. FIG. 19 is a block diagram of an endoscope surgical robot system according to an additional exemplary embodiment, FIG. 20 is a perspective view of an operation module according to an additional exemplary embodiment, and FIG. 21 is a view illustrating an output image of a display part according to an operation of an operation module according to an additional exemplary embodiment and an operation configuration of an endoscope apparatus.

Referring to FIGS. 18 to 21, an endoscope surgical robot system 8 according to the additional exemplary embodiment receives an input signal from a fourth operation module 82 corresponding to a master device which generates and transmits an input signal required for the manipulation of the user to drive the insertion tube 111 of the endoscope apparatus 11 by means of a slave device which is directly connected to the endoscope apparatus 11 to perform an operation corresponding to the input signal.

For example, the endoscope surgical robot system 8 may include an endoscope apparatus 11, a driving module 13, a fourth operation module 82, a display part 14, and a controller 85.

The endoscope apparatus 11 may include an insertion tube 111 which is inserted into a body of a patient to perform the translation and rotation and an endoscopic camera 112 which photographs an image at an end portion of the insertion tube 111.

For example, the endoscope apparatus 11 may be connected to the driving module 13 and the driving module 13 may be driven to perform the translation and the rotation of the insertion tube 111.

The driving module 13 may be connected to the endoscope apparatus 11 to perform the translation and the rotation of the insertion tube 111. For example, the driving module 13 may be a slave device which receives an input signal from the fourth operation module 82 corresponding to a master device to directly perform the operation required for the endoscope apparatus 11.

For example, the driving module 13 may include a translation driving part 131 which performs translation P_translation of the insertion tube 111 along a translation movement axis X_T with respect to a length direction of the insertion tube 111, a roll driving part 132 which performs roll rotation R_roll around a roll rotation axis X_R parallel to the length direction of the insertion tube 111, and a pitch driving part 133 which performs pitch rotation R_pitch to bend the insertion tube 111 with respect to a pitch rotation axis X_P perpendicular to the roll rotation axis X_R.

The fourth operation module 82 may be an input device manipulated by the user. For example, the fourth operation module 82 may be a master device which is operated by the user to generate an input signal required for driving the endoscope apparatus 11.

For example, the fourth operation module 82 may include a translation movement part 821, a roll rotation part 822, a pitch rotation part 823, and a control handle 824.

The translation movement part 821 may slidably move along the translation movement axis X_T corresponding to a forward/backward direction of the insertion tube 111 in a state in which the user grasps the control handle 824 and thus, the controller 85 may control the operation of the translation driving part 131 which performs the forward/backward operation of the insertion tube 111 based on a translation movement displacement of the translation movement part 821.

For example, the translation movement part 821 may include a sliding guide 8211 extending along the translation movement axis X_T and a sliding arm 8212 which is guided by the sliding guide 8211 to slide along the translation movement axis X_T.

The roll rotation part 822 may rotate the joint along the roll rotation axis X_R corresponding to a rotation axis of the roll rotation R_roll of the insertion tube 111 in a state in which the user grasps the control handle 824. Accordingly, the controller 85 may control the operation of the roll driving part 132 which performs the roll rotation R_roll of the insertion tube 111 based on the rotation displacement of the roll rotation part 822.

For example, the roll rotation part 822 may include a roll rotation arm 8221 which rotates a joint around the roll rotation axis X_R extending parallel with the translation movement axis X_T with respect to the sliding arm 8212 and a first rotation encoder 8222 which measures a rotation movement of the roll rotation arm 8221.

In the meantime, as another example, in the fourth operation module 82, a configuration of a translation movement part 821 may be omitted. In this case, it is noted that the roll rotation arm 8221 of the roll driving part 832 may be rotatably installed in an arbitrary member connected from a fixed base point of the fourth operation module 82.

The pitch rotation part 823 may rotate the joint along the pitch rotation axis X_P corresponding to a rotation axis of the pitch rotation R_pitch of the insertion tube 111 in a state in which the user grasps the control handle 824. Accordingly, the controller 85 may control the operation of the pitch driving part 133 which performs the pitch rotation R_pitch of the insertion tube 111 based on the rotation displacement of the pitch rotation part 823.

For example, the pitch rotation part 823 may include a pitch rotation arm 8231 which rotates a joint around the pitch rotation axis X_P perpendicular to the roll rotation axis X_R with respect to the roll rotation arm 8221 and a second rotation encoder 8232 which measures a rotation movement of the pitch rotation arm 8231.

The control handle 824 may be a member which is connected from the pitch rotation part 823 to be grasped by the user. The user may move or rotate at least one of the translation movement part 821, the roll rotation part 822, and the pitch rotation part 823 while grasping the fourth control handle 824.

For example, a control handle 824 may provide an input interface to control operations of the endoscope apparatus 11 or various surgical tools used together.

As illustrated in FIG. 20, the fourth operation module 82 may have a joint link structure in which the sliding arm 8212, the roll rotation arm 8221, the pitch rotation arm 8231, and the control handle 824 are sequentially and relatively inwardly bent to be connected while being spaced apart from one another.

The display part 14 may receive and output an image generated from the endoscope camera 112. For example, the controller 85 may receive an image generated by the endoscope camera 112 and transmit the image to the display part 14 to output the image.

The controller 85 may receive the input signal generated by the fourth operation module 82 manipulated by the user and control the operation of the driving module 13 based on the input signal to drive the endoscope apparatus 11 in response to the input signal.

For example, the controller 85 may control the roll rotation R_roll of the insertion tube 111 based on a signal measured by the first rotation encoder 8222 of the roll rotation part 822 and control the pitch rotation R_pitch of the insertion tube 111 based on a signal measured by the second rotation encoder 8232 of the pitch rotation part 823.

For example, during the process of manipulating the fourth operation module 82 by the user, when the roll rotation part 822 rotates around the roll rotation axis X_R, the controller 85 may perform a correction to rotate an image output on the display part 14 together in proportion to a direction of the rotation and the rotation displacement.

In other word, the controller 85 may calculate a direction and a displacement of the roll rotation arm 8221 of the roll rotation part 822 rotated by the user through a signal measured by the first rotation encoder 8222 of the roll rotation part 822 and correct the image input from the endoscope camera 112 to be rotated in accordance with the rotation direction and the rotation displacement of the roll rotation arm 8221, and transmit and output the rotated and corrected image to the display part 14.

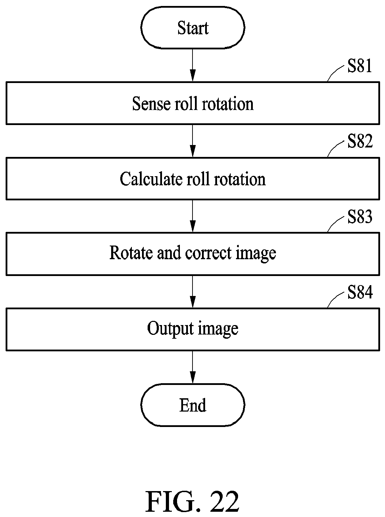

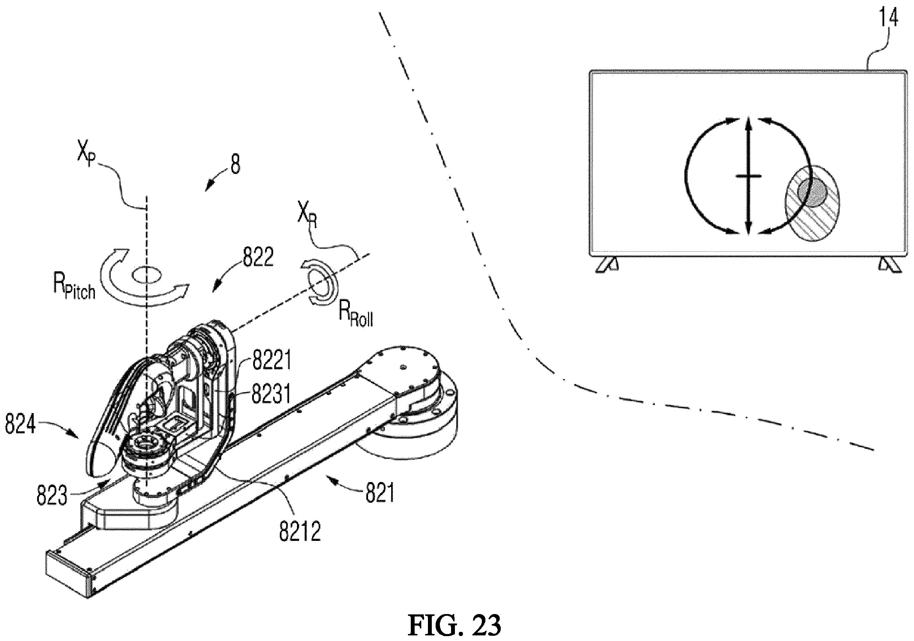

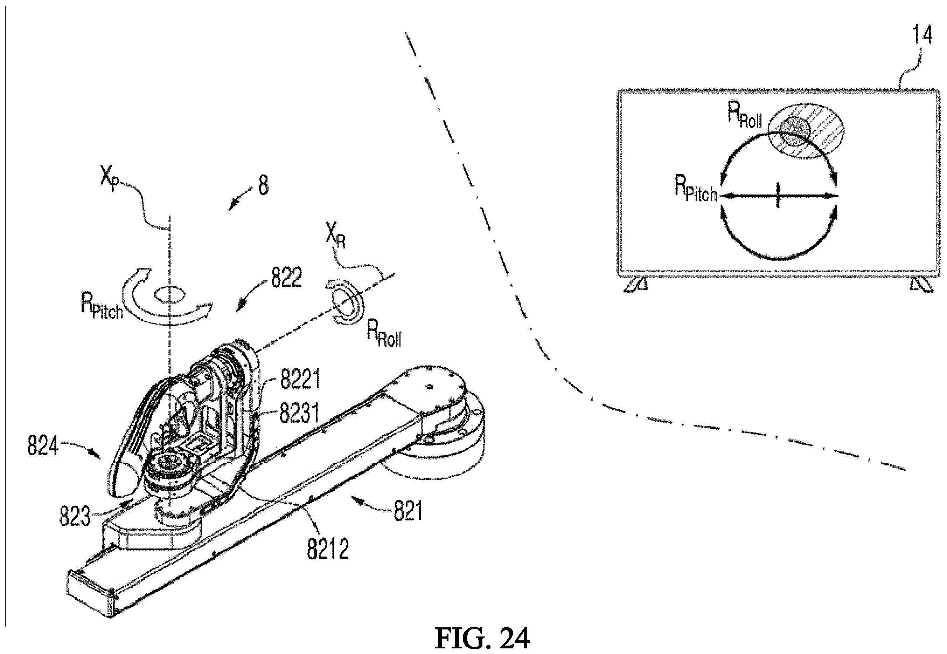

FIG. 22 is a flowchart of a method for correcting an output image of an endoscope surgical robot system according to an additional exemplary embodiment, FIG. 23 is a view illustrating a configuration of an output image of a display part according to an operation of an operation module according to an additional exemplary embodiment, and FIG. 24 is a view illustrating a configuration of an output image of a display part according to an operation of an operation module according to an additional exemplary embodiment.

Referring to FIGS. 22 to 24, a method for correcting an output image of an endoscope surgical robot system 8 which generates an input signal of a driving module 13 of an endoscope apparatus 11 through a fourth operation module 82 in which the pitch rotation part 823 is located prior to the roll rotation part 822 with respect to the control handle 824 grasped by a user may be identified.

In the meantime, it is noted that in FIG. 23, the image output through the display part 14 corresponds to an image which has not been corrected by the method of the present exemplary embodiment and is provided for comparison with FIG. 24 in which a corrected image is output.

The method for correcting an output image according to an additional exemplary embodiment may be described with a configuration of an endoscope surgical robot system 8 according to the exemplary embodiment of FIGS. 18 to 21. However, it is merely illustrative and it is noted that it may be applied to all the arbitrary endoscope surgical robot systems including a master device (an operation module) which forms a joint at a point where a configuration of a pitch rotation part which rotates to input a pitch rotation operation of the insertion tube is closer to an end grasped by the user than a configuration of a roll rotation part which rotates to input roll rotation operation of the insertion tube.

The method for correcting an output image of an endoscope surgical robot system according to an additional exemplary embodiment may include a step S81 of sensing a roll rotation movement of a roll rotation part 822 by a controller 85 during a process of manipulating a fourth operation module, by a user, a step S82 of calculating roll rotation movement information including a direction and a displacement of the roll rotation part 822 rotated by the user based on the sensed roll rotation movement by the controller 85, a step S83 of rotating and correcting an image which is generated and transmitted by the endoscope camera 112 based on the calculated roll rotation movement information by the controller 85, and a step S84 of transmitting and outputting a corrected image to the display part 15 by the controller 85.

In the step S81 of sensing a roll rotation movement, the controller 85 may sense the rotation of the roll rotation part 822 around the roll rotation axis X_R through the signal measured by the first rotation encoder 8222 of the roll rotation part 822.

For example, the controller 85 may perform the step S82 of calculating the roll rotation movement information through the signal applied from the first rotation encoder 8222.

In the step S82 of calculating roll rotation movement information, the controller 85 may calculate the roll rotation movement information including a direction and a displacement of the roll rotation arm 8221 of the roll rotation part 822 which is rotated around the roll rotation axis X_R by the user based on the signal measured by the first rotation encoder 8222 of the roll rotation part 822.

In the step S83 of rotating and correcting the image, the controller 85 may rotate an image generated by the endoscope camera 112 in the same direction or rotate the image at an angle proportional to the rotation displacement, based on the rotation direction and the rotation displacement of the roll rotation arm 8221 included in the roll rotation movement information.

For example, the controller 85 may rotate the image generated by the endoscope camera 112 at the same angle as the rotation displacement of the roll rotation arm 8221.

According to the endoscope surgical robot system 8 according to an exemplary embodiment and an image correcting method thereof, as illustrated in FIGS. 23 and 24, it is understood that when the user rotates the control handle 824 around the roll rotation axis X_R, the pitch rotation part 823 also rotates together so that the pitch rotation axis X_P of the pitch rotation part 823 also rotates around the roll rotation axis X_R.

Here, as illustrated in FIG. 23, when the image generated by the endoscope camera 112 is output to the display part 14 without being corrected, a pitch rotation R_pitch direction on the output image coordinate and an actual rotation direction of the pitch rotation part 823 do not match, which may cause manipulation confusion to the user who manipulates the fourth operation module 82 while watching the display part 14.

However, as illustrated in FIG. 24, when the image which is rotated and corrected in accordance with the rotation of the roll rotation part 822 by the method for correcting an output image according to the exemplary embodiment is output to the display part 14, the pitch rotation R_pitch direction on the output image coordinate and an actual rotation direction of the pitch rotation part 823 are set to be identical, which allows the user to intuitively manipulate the fourth operation module 82.

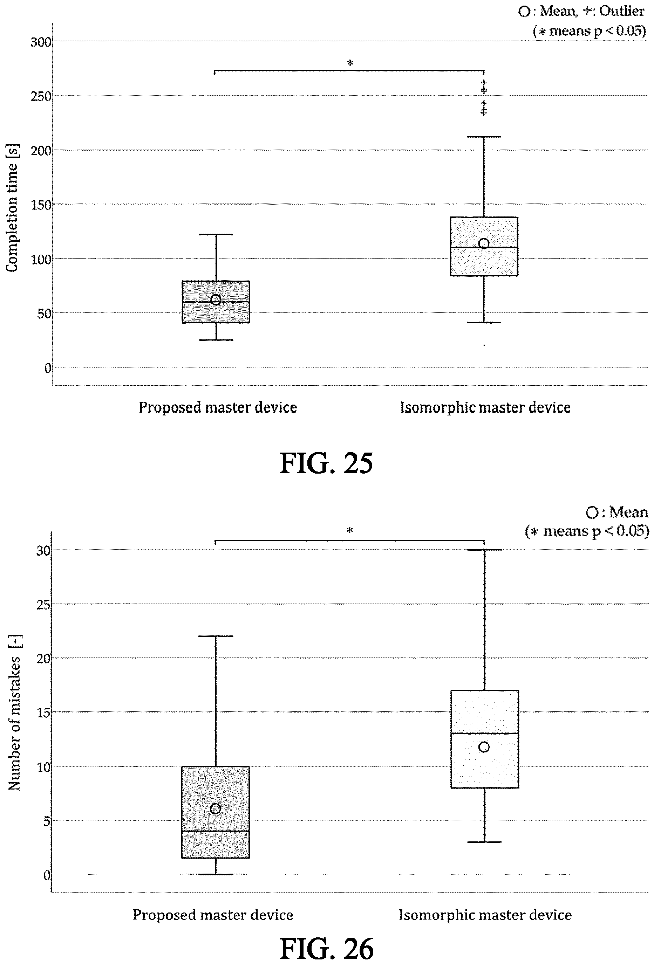

FIG. 25 is a graph to compare surgery time taken when using a conventional master device for an endoscope surgical robot and surgery time taken when using a master device according to an exemplary embodiment for an endoscope surgical robot.

FIG. 25 shows a time difference between the case where an operation was performed using the master device for the endoscope surgical robot according to the above-described embodiments and the case where the same operation was performed using the conventional master device (shown in FIGS. 1A and 1B).

When using the master device described in the present disclosure, it takes an average of 61.93 seconds, whereas when using the conventional master device, it takes an average of 114.49 seconds. As such, the master device described in the present disclosure enables faster surgery than the conventional master device.

FIG. 26 is a graph to compare mean number of mistakes occurred when using a conventional master device for an endoscope surgical robot and mean number of mistakes occurred when using a master device according to an exemplary embodiment for an endoscope surgical robot.

FIG. 26 shows that the mean number of mistakes is 6.07 when using the master device for an endoscopic surgical robot according to the embodiments of the present disclosure, and the mean number of mistakes is 12.80 when using the conventional master device.

As such, the master device described in the present disclosure enables more accurate surgery than the conventional master device.

The present disclosure described as above is not limited by the aspects described herein and accompanying drawings. It should be apparent to those skilled in the art that various substitutions, changes and modifications which are not exemplified herein but are still within the spirit and scope of the present disclosure may be made. Therefore, the scope of the present disclosure is defined not by the detailed description, but by the claims and their equivalents, and all variations within the scope of the claims and their equivalents are to be construed as being included in the present disclosure.

## Claims

1. An endoscope surgical robot control device that controls the movement of an insertion tube, the control device comprising:
a translation movement part configured to control translation movement of the insertion tube in a translation direction;
a pitch rotation part in communication with the translation movement part and configured to control pitch rotation of the insertion tube around a pitch rotation axis; and
a roll rotation part in communication with the pitch rotation part and configured to control roll rotation of the insertion tube around a roll rotation axis which is perpendicular to the pitch rotation axis.

2. The device of claim 1, wherein the translation movement, the pitch rotation and the roll rotation are operated independently from one another.

3. The device of claim 1, wherein the translation movement part is in direct communication with the pitch rotation part, and the pitch rotation part is in direct communication with the roll rotation part.

4. The device of claim 1, wherein the translation movement part comprises:
a sliding guide extending along the translation direction in a forward and backward direction; and
a translation sliding part, one end of which is configured to slide along the sliding guide and the other end of which has a sliding arm which extends upward and is connected to the pitch rotation part.

5. The device of claim 4, wherein the pitch rotation part includes:
the pitch rotation arm which is connected to the sliding arm and is configured to rotate around the pitch rotation axis; and
a handle having a first end and a second end, the first end connected to the pitch rotation arm and the second end connected to the roll rotation part.

6. The device of claim 5, wherein the roll rotation part comprises:
a roll rotation arm connected to the handle and configured to rotate around the roll rotation axis.

7. The device of claim 4, wherein the handle comprises:
a first fine adjustment wheel configured to control fine translation movement of the insertion tube; and
a second find adjustment wheel configured to control fine pitch rotation of the insertion tube.

8. The device of claim 4, wherein the handle comprises:
a joystick configured to move in at least four directions to control fine translation movement and fine pitch rotation of the insertion tube,
wherein movement of the joystick in an up and down direction controls the fine translation movement, and movement of the joystick in a left and right direction controls the fine pitch rotation,
the up and down direction being parallel with the roll rotation axis, and
the left and right direction being parallel with the pitch rotation axis.

9. The device of claim 1, further comprising:
a basket controller configured to control a basket connected to the basket controller through a link within the insertion tube,
wherein the basket controller comprises:
a translation movement actuator configured to control translation movement of the basket; and
an open and close actuator configured to control open and close operation of the basket.

10. The device of claim 1, further comprising:
a forceps controller configured to control forceps connected to the forceps controller through a link within the insertion tube,
wherein the forceps controller controls translation movement of the forceps and an open and close operation of the forceps.

11. The device of claim 1, further comprising:
a laser controller configured to control a laser generation device within the insertion tube,
the laser controller controls translation movement of the laser generation device and operation of the laser generation device.

12. The device of claim 1, wherein the translation movement part is configured to control movement of the insertion tube in a forward and backward direction, and
wherein the insertion tube is configured to move di/k when the translation movement part moves di, k being positive integer.

13. The device of claim 1, wherein the pitch rotation part is configured to rotate around the pitch rotation axis, and
wherein torque required for rotation of the pitch rotation part is configured to change depending on a distance between a side of the insertion tube and an inner wall of an organ.

14. The device of claim 1, wherein the roll rotation part is configured to rotate around the roll rotation axis, and
wherein a torque required for rotation of the roll rotation part is configured to change depending on curvature of the insertion tube.

15. An endoscope surgical robot system, comprising:
an endoscope apparatus having an insertion tube and an endoscope camera to monitor a surgical procedure where the insertion tube is inserted;
a driving module connected to the endoscope apparatus and configured to perform pitch rotation or roll rotation of the endoscope apparatus;
the endoscope surgical robot control device of claim 1; and
a controller to control operation of the driving module based on an input signal generated by the endoscope surgical robot control device.
